# EUROPÄISCHE PATENTSCHRIFT

(11) **EP 4 110 531 B1**
(45) Veröffentlichungstag und Bekanntmachung des Hinweises auf die Patenterteilung: **25.03.2026**
(21) Anmeldenummer: 20740210.8
(22) Anmeldetag: 08.06.2020
(51) Int. Cl.: B05B 17/00, A61M 11/00, A61M 15/00

(54) **AEROSOLERZEUGER IN SANDWICH-BAUWEISE**
AEROSOL GENERATOR HAVING A SANDWICH CONSTRUCTION
GÉNÉRATEUR D'AÉROSOL À CONSTRUCTION EN SANDWICH

(43) Veröffentlichungstag der Anmeldung: 04.01.2023
(73) Patentinhaber: NEBU-TEC med. Produkte Eike Kern GmbH, 63820 Elsenfeld (DE)
(72) Erfinder: KERN, Joachim, 63820 Elsenfeld (DE)
(74) Vertreter: Scharfenberger, Burkhard
(86) Internationale Anmeldenummer: PCT/DE2020/100481
(87) Internationale Veröffentlichungsnummer: WO 2021/249585

(56) Entgegenhaltungen:
- DE-U1- 202009 008 436
- US-A1- 2003 192 956
- US-A1- 2011 233 302

## Beschreibung

Die vorliegende Erfindung betrifft einen Aerosolerzeuger gemäß dem Oberbegriff des Anspruchs 1 sowie ein Verfahren zu dessen Herstellung.

Vernebler dienen der Aerosolisierung von Fluiden, d.h. der Umwandlung eines größeren, zusammenhängenden Fluidvolumens in einen Nebel aus mehr oder weniger feinen Fluidtröpfchen. Sie werden insbesondere bei der Therapie von Atemwegs- und Lungenerkrankungen eingesetzt, wobei hierfür das zu aerosolisierende Fluid üblicherweise eine wässrige Wirkstofflösung ist. Die angestrebte Größenverteilung der Aerosol-Tröpfchen ist dabei abhängig von dem gewünschten Depositionsort. Eine Behandlung von Bronchialerkrankungen erfordert Tröpfchengrößen von 5 Mikrometer und mehr, wohingegen bei einer gewünschten Deposition des Wirkstoffes in den Lungenbläschen die Tröpfchengrößen möglichst unter 5 Mikrometer liegen sollte.

Das der Verneblung oder Aerosolisierung des Fluides dienende Herzstück eines Verneblers ist der eigentliche Aerosolerzeuger. Heutzutage werden hierfür weitgehend Aerosolerzeuger vom Mesh-Membran-Typ eingesetzt. Bei diesem Typ Aerosolerzeuger ist ein in einem zentralen Bereich perforiertes und üblicherweise kalottenartig gebogenes Plättchen von rundem Grundriss, die Mesh-Membran, auf einer Trägerscheibe befestigt, welche durch eine Piezokeramik in schnelle Schwingung versetzt wird. Hierdurch wird das auf einer Seite der Mesh-Membran an diese anliegende Fluid in die Poren der Perforation gedrückt und auf der gegenüberliegenden Auslassseite als Aerosol, also als ein mehr oder weniger feiner Nebel aus Fluidtröpfchen abgegeben. Der Aerosolerzeuger wird im Inneren des Verneblergehäuses in seiner Arbeitsposition festgehalten, indem er zwischen zwei annähernd torioidaler Gummiringen bzw. -lippen aus einem möglichst weichen Gummi eingeklemmt wird.

Parameter, welche die Tröpfchengrößenverteilung beeinflussen, sind der Durchmesser und Geometrie der Poren, die Schwingungsfrequenz, welche durch die Frequenz der angelegten Spannung bestimmt wird, die Shore-Härte und die Angriffspunkte der den Aerosolerzeuger einklemmenden Gummiringe- oder lippen sowie die sich aufgrund der Geometrie und des Aufbaus des Aerosolerzeugers ergebende Raumform der stehenden Schwingung der Mesh-Membran. Bei letzterem spielt zum eine Rolle, ob die Piezokeramik unmittelbar oder nur mittelbar über ein zwischengeschaltetes Element mit der Mesh-Memban verbunden ist, welche Dicke und Form die Mesh-Membran aufweist und in welcher Weise die Membran mit der Piezokeramik bzw. dem Zwischenelement verbunden ist.

Ebenfalls von großem Einfluss ist die Geometrie der Mesh-Membran. Hier hat sich im Stand der Technik Mesh-Membranen mit einem runden Grundriss und einer kalottenartigen, konvexen Wölbung im perforierten Bereich durchgesetzt.

Bei heute eingesetzten Aerosolerzeugern vom beschriebenen Mesh-Membran-Typ ist der übliche Aufbau der, dass auf einer Oberseite einer Scheibe aus (Edel-)Stahl, welche kreisringförmig ist und also eine konzentrische, durchgängige Öffnung aufweist, die Mesh-Membran angebracht, während auf der gegenüberliegenden Unterseite der Scheibe die anregende, ebenfalls kreisringförmige Piezokeramik befestigt wird. Die Befestigung erfolgt hierbei im Stand der Technik bevorzugt durch Verkleben der Komponenten mittels eines für medizinische Zwecke zugelassenen Klebstoffes. Ein solcher Klebstoff darf beim Kontakt mit einer wässrigen Wirkstofflösung keine gesundheitsschädlichen Stoffe an diese Lösung abgeben.

Um sie mit solchen verträglichen Klebstoffen verkleben zu können, muss die Mesh-Membran aus einem entsprechenden Material bestehen. Im Stand der Technik werden daher üblicherweise Edelstahlplättchen als Mesh-Membran eingesetzt, da diese sowohl mit Edelstahl-Zwischenscheiben als auch direkt auf die Keramik geklebt werden können.

Der Nachteil von Mesh-Membranen aus Edelstahl, ist jedoch ihre vergleichsweise aufwendige und daher kostspielige Herstellung.

Deutlich günstiger weil einfach parallelisierbar wäre eine elektrogalvanische Herstellung, bei der Mesh-Membranen der gewünschten Form inklusive der Perforierung in einem entsprechend geformten Negativ elektro-galvanisch abgeschieden werden. Hierdurch lassen sich große Stückzahlen schnell und einfach herstellen. Diese Methode ist mit der Verwendung von Edelstahl oder anderen Metalllegierungen jedoch nicht vereinbar. Das Material der Wahl für eine elekrogalvanische Herstellung ist demgegenüber Nickel.

Ein weiteres zukünftig interessante Materialklasse für Mesh-Membranen sind Kunststoffe, insbesondere eines Fluor-terminierte Kunststoff. Diese bieten den Vorteil, dass sie eine hydrophobe Oberfläche besitzen, wodurch sie durch die üblicherweise verwendeten wasserbasierten Wirkstofflösungen schlechter benetzt werden, was der Bildung der feinerer Tröpfchen dienlich ist und die Anhaftung herabsetzt und damit der bei metallenen Mesh-Membranen schwer zu vermeidenden Akkumulation von Fluid, welches nicht mit ausreichender kinetischer Energie die Poren verlassen hat, auf der Auslassseite entgegenwirkt.

Mesh-Membranen aus Nickel wie auch aus Fluor-terminiertem Kunststoff ist allerdings gemeinsam, dass sie sich nicht mit der Trägerscheibe des Aerosolerzeugers verkleben lassen oder zumindest nicht mittels eines für den Kontakt mit Wirkstoffflüssigkeiten zugelassenen Klebstoffes. Dies verhinderte bisher ihren, wie dargelegt durchaus wünschenswerten, Einsatz in Aerosolerzeugern.

Die Offenlegungsschrift US 2011/0233302 A1 schlägt einen Aerosolerzeuger aus einer Mesh-Membran und einer auf einer Oberseite aufgeklebten piezoelektrischen Antriebselement, wobei die Mesh-Membran eine zentrale Verneblungsregion mit Düsen und ein radial weiter außen gelegene Kleberegion mit Klebelöchern vorsieht. Wird eine Klebstoffschicht über der Kleberegion appliziert, fließt der Klebstoff in die Klebelöcher, was nach dem Aushärten für eine sehr feste Verbindung zwischen der getrockneten Klebstoffschicht und der Mesh-Membran ermöglicht. Hinsichtlich der Größe oder Form der Klebelöcher in absoluter Hinsicht und/oder relativ zur Größe der Düsen macht die Druckschrift keine expliziten Angaben, offenbart jedoch in den Figuren Klebelöcher von der gleichen Größenordnung und (kreisrunden) Form, wie die Düsen.

Die Gebrauchsmusterschrift DE 20 2009 008 436 U1 offenbart eine Vorrichtung zur Tröpfchenbildung umfassend eine Düsenscheibe, welche zwischen einem Verbundplättchen und einem Schwingungsplättchen, welche beide kreisringförmig sind, eingebettet und durch Verkleben befestigt ist. Um die Klebeverbindung zu verbessern, verfügt die Düsenscheibe in ihrem Randbereich um kreisringsegmentförmige durchgehende Öffnungen, mittels derer sich Klebstoffschichten auf beiden Seiten der Düsenscheibe in Kontakt treten und verbinden können. Konkret werden vier symmetrisch angeordnete durchgehende Öffnungen vorgeschlagen. Die vorliegende Erfindung hat sich vor diesem Hintergrund die Aufgabe gestellt, bei Aerosolerzeugern mit nichtverklebbarer Meshmembranen die Erzeugung feinerer Aerosoltröpfchen zu ermöglichen .

Diese Aufgabe wird auf elegante Art durch einen Aerosolerzeuger nach Anspruch 1 gelöst, welcher gemäß Anspruch 10 hergestellt werden kann.

Bei dem erfindungsgemäßen Aerosolerzeuger wird die Mesh-Membran sicher befestigt, ohne dafür die Membran selbst zu Verkleben nötig, indem umfänglich des Außenrandes der Membran von der Ober- zur Unterseite durchgängige Öffnungen, hier als Verklebeöffnungen bezeichnet, eingefügt werden. Durch diese Verklebeöffnungen ragen im fertigen Aerosolerzeuger
säulenartige Strukturen aus erhärtetem Klebstoff, welche eine oberhalb und unterhalb der Membran gelegene Klebstoffschicht miteinander verbinden. Die beiden Klebstoffschichten sind jeweils mit einer unterhalb der Mesh-Membran gelegenen Trägerstruktur oder -scheibe bzw. einem oberhalb der Mesh-Membran gelegenen Abschlussring verklebt. Dadurch wird die Membran fest und unbeweglich gehalten, ohne dass sie selbst verklebt ist.

Ein solcher erfindungsgemäßer Aersolerzeuger wird aus den bereitgestellten Komponenten Mesh-Membran, Abschlussring und der die Piezokeramik umfassenden Trägerstruktur mit Öffnung derart hergestellt, dass zunächst umfänglich der, üblicherweise einen kreisrunden Grundriss aufweisenden Mesh-Membran entlang ihres Außenrandes eine gewünschte Anzahl Verklebeöffnungen in einem gewünschten Muster eingebracht werden, etwa durch Bohren. Alternativ und bevorzugt würde die Mesh-Membran bereits in der gewünschten Konfiguration hergestellt. Weiterhin wird auf die Oberseite der Trägerstruktur ein hochviskoser, aber noch ausreichend fließfähiger Klebstoff aufgebracht, etwa als etwa ringförmige, die Öffnung umschließende Formation . Diese Klebstoffformation bzw. der Klebstoffring sollte ein Volumen beinhalten, welches dem Gesamtvolumen aller Verklebeöffnungen der Mesh-Membran entspricht (wobei man sich bei der Bestimmung des Volumens einer Klebeöffnung Ober und- Unterseite durch ebene Flächen verschlossen denken muss) zuzüglich einer Menge die zur Ausbildung zweier Klebstoffschichten einer gewünschten Dicke zischen Membran und Trägerstruktur bzw. Membran und Abschlussring ausreicht.

Sodann setzt man die Mesh-Membran derart auf die Trägerstruktur auf, dass die Öffnung der Trägerstruktur in gewünschter weise 6 bedeckt, ist, insbesondere konzentrisch zu der üblicherweise runden Öffnung, und presst sie soweit an, wobei Klebstoff in die Verklebeöffnungen hinein und durch sie hindurch gepresst wird, dass sich zwischen Trägerstruktur und Mesh-Membran eine gleichmäßige Klebstoffschicht der gewünschten Dicke ausbildet. Die Klebstoffmenge ist, wie oben angegeben, so bemessen, dass das Klebstoffvolumen die Verklebeöffnungen der Mesh-Membran nicht nur ganz ausfüllt, sondern darüber hinaus quillt und über die Oberseite der Mesh-Membran herausragende Klebstoffpunkte bildet. Auf diese Oberseite wird nun der kreisringförmige Abschlussring aufgelegt und so festgedrückt, dass sich eine möglichst gleichmäßige Klebstoffschicht gewünschter Stärke ausbildet, wobei etwaig überschüssiger Klebstoff über den Rand des Rings hinausquillt. Dieser wird bevorzugt entfernt, um eine negative Beeinflussung der Schwingungseigenschaften der Membran zu vermeiden. Das Entfernen erfolgt entweder direkt, d.h. solange der Klebstoff noch flüssig ist, oder, um das Risiko einer ungewollten Verschiebung der Komponenten zu vermeiden, in einem Nachbehandlungsschritt nach dem Aushärtenlassen.

Durch die erfindungsgemäße Art der Befestigung mittels des beschriebenen sandwichartigen Aufbaus aus Trägerstruktur - Mesh-Membran - Abschlussring mit jeweils dazwischenliegender Klebeschicht kann eine Mesh-Membran aus nicht verklebbarem Material in einem Aerosolerzeuger verwendet werden. Hierdurch würden entweder die Herstellungskosten des Aerosolerzeugers gesenkt, etwa bei Verwendung einer elektrogalvanisch hergestellten Mesh-Membran aus Nickel und/oder die Tröpfchengrößenverteilung positiv beeinflusst, etwa bei Verwendung einer Mesh-Membran aus Kunststoff, insbesondere Fluor-terminierten Kunststoff.

7
Als weiterer Vorteil der erfindungsgemäßen Befestigungsart hat sich herausgestellt, dass sich auch schon bei Nickel-Membranen eine kleine mittlere Tröpfchengröße und eine lungengängigere Verteilung der Tröpfchengrößen einstellt. In Versuchen wurden Tröpfchengrößenverteilungen gemessen, bei welchen über 80% der Masse des Aerosols in Tröpfchen mit Durchmesser unter 5 Mikrometern vorlag. Dies stellte eine deutliche Steigerung gegenüber gleichartig geformten Edelstahl-Mesh-Membranen dar, welche auf herkömmliche Art durch direktes Verkleben mit der Trägerstruktur befestigt waren.

Bisher ist nicht vollständig geklärt, wie die Befestigung diesen positiven Einfluss ausüben kann. Es kann jedoch spekuliert werden, dass die Krafteinleitung über eine Reihe diskreter Punkte, nämlich über die die Verklebeöffnungen durchgreifenden Klebstoffsäulen, welche gleichmäßig über den Umfang der Mesh-Membran verteilt sind, möglicherweise eine gleichförmigere und symmetrischere Form der stehenden räumliche Wellenform der Mesh-Membran bewirkt, als dies bei einer vollflächigen Verklebung der Fall ist.

Im Weiteren werden weitere vorteilhafte Ausführungen vorliegender Erfindung vorgestellt, welche in geeigneter Form miteinander kombiniert werden können, sofern sie sich nicht gegenseitig offensichtlich ausschließen.

Obwohl sich der Einsatzbereich der vorliegenden Erfindung nicht darauf einschränkt, ist die im erfindungsgemäßen Aerosolerzeuger eingesetzte Mesh-Membran bevorzugt aus einem Material hergestellt, welches nicht mittels eines für medizinische Einsatzzwecke, insbesondere den Kontakt mit Wirkstoffflüssigkeiten zugelassenen Klebstoffes verklebbar ist. Besonders bevorzugt wird eine Mesh-Membran aus Nickel oder einem Kunststoff, insbesondere einem mit Fluor-Kohlenstoff Verbindungen enthaltenden Kunststoff eingesetzt.

Die erfindungsgemäßen Verklebeöffnungen der Mesh-Membran sind bevorzugt mehr- oder vielzählig und/oder gleichmäßig und/oder alle oder gruppenweise in gleichem Abstand zum Rand der Mesh-Membran angeordnet. Besonders bevorzugt werden die Verklebeöffnungen in einem oder zwei konzentrisch um einen Mittelpunkt der Mesh-Membran angeordneten Ringen angeordnet, wobei jeder der Ringe drei oder mehr gleichverteilte Verklebeöffnungen aufweist. Die Verklebeöffnungen sind bevorzugt rund oder quadratisch mit einem Radius bzw. einer Kantenlänge von zwischen 0,1 und 1 Millimeter und haben einen Abstand zum Außenrand der Mesh-Membran, welcher mindestens dem Anderthalbfachen ihres Radius bzw. ihrer halben Kantenlänge entspricht.

Die Trägerstruktur des erfindungsgemäßen Aerosolerzeugers ist in einer Ausführungsform vorliegender Erfindung eine runde Stahlscheibe mit einer konzentrischen durchgängigen Öffnung in der Mitte, wobei an der der Mesh-Membran abgewandten Unterseite der Scheibe die zur Schwingungsanregung nötig Piezokeramik angebracht, insbesondere angeklebt, ist.

In einer alternativen Ausführungsform vorliegender Erfindung ist die Trägerstruktur eine kreisringförmige Piezokeramik. Hierdurch wird eine größere Schwingungsamplitude und damit -energie erreicht, was für die Verneblungsrate sowie die Tröpfchengrößenverteilung vorteilhaft ist. Weiterhin kann ein Herstellungsschritt eingespart werden, da das Befestigen, insbesondere Verkleben, der Piezokeramik auf einer Stahlscheibe entfallen kann.

Als Klebstoff zur Befestigung aller Teile des erfindungsgemäßen Aerosolerzeugers wird bevorzugt ein Epoxidharz eingesetzt. Dieses ist einfach in der Handhabung, medizinverträglich, kann leicht auf die gewünschte Viskosität - fest genug um zwischen Auftragen und Festpressen der Mesh-Membran nicht wegzufließen aber flüssig genug um leicht in die Verklebeöffnungen einzudringen - eingestellt werden und härtet recht schnell aus.

Weitere Eigenschaften, Merkmale und Vorteile vorliegender Erfindung ergeben sich aus der im Folgenden anhand der Figuren näher erläuterten, beispielhaften Ausführungen. Diese sollen vorliegende Erfindung nur illustrieren und in keiner Weise einschränken.

Es zeigen:
- Figur 1:: Ein Querschnitt durch eine Ausführungsform eines erfindungsgemäßen Aerosolerzeugers
- Figur 2:: Zwei mögliche Ausführungsformen einer Mesh-Membran eines erfindungsgemäßen Aerosolerzeugers in Draufsicht
- Figur 3:: Eine Illustration der Herstellung eines erfindungsgemäßen Aerosolerzeugers in drei Teilfiguren

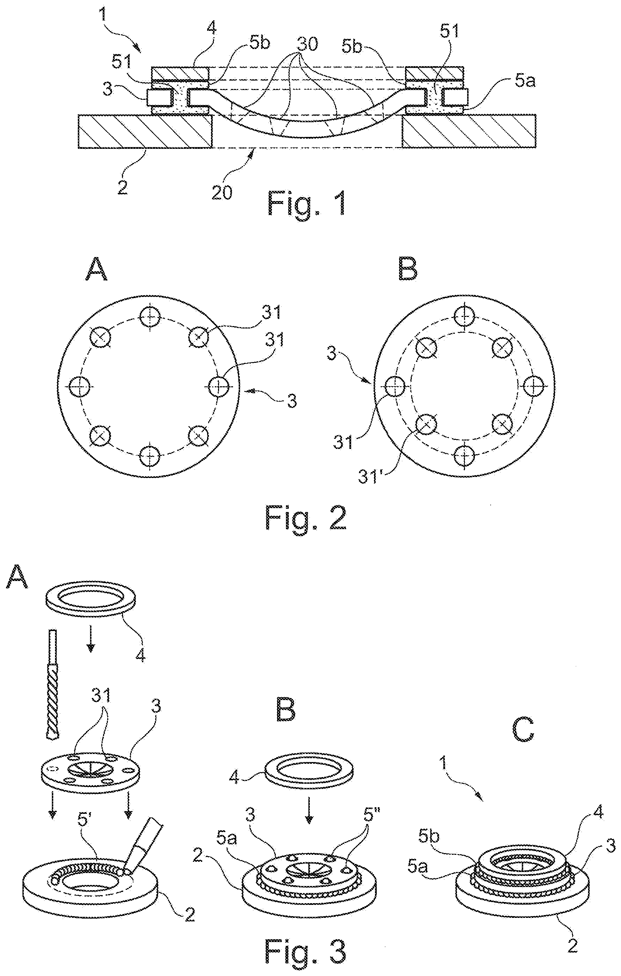

In **Figur 1** ist ein Querschnitt durch eine mögliche Ausführungsform eines erfindungsgemäßen Aerosolerzeugers dargestellt.

Auf der Oberseite der Trägerstruktur 2 sind die Mesh-Membran 3 und darüber wiederum der Abschlussring 4 aufgesetzt. Die Mesh-Membran 3, welche wie dargestellt einen ebenen, kreisringförmigen Randbereich und einen konvex gebogenen, mit trichterförmigen Poren versehenen Zentralbereich aufweist, hat im flachen Außenbereich eine Mehrzahl umfänglich angeordneter, von Ober- zur Unterseite durchgängiger Verklebeöffnungen, welche jeweils von säulenartigen Strukturen 51 aus ausgehärtetem Klebstoff durchgriffen werden. Die Klebstoffsäulen 51 verbinden eine untere Klebstoffschicht 5a mit einer oberen Klebstoffschicht 5b und bilden somit eine im Querschnitt wie ein auf der Seite liegendes H aussehende Klebstoffstruktur. Die Klebstoffschichten 5a, 5b sind mit den jeweils unter- bzw. oberhalb angrenzenden, aus einem zur Verklebung geeigneten Material gefertigten Komponenten verklebt, nämlich der Trägerstruktur 2 im Falle der unteren Klebstoffschicht 5a und dem Abschlussring 4 im Falle der oberen Klebstoffschicht 5b. Um eine möglichst harmonische Schwingungsverhalten des Aerosolerzeugers zu garantieren haben die Klebstoffschichten eine möglichst gleichförmige Stärke. Aus gleichem Grund werden bei der erfindungsgemäßen Herstellung seitlich über den Aussenring 4 oder die Mesh-Membran 3 hervorgequollene überschüssige Klebstoffanteile bevorzugt entfernt.

**Figur 2** zeigt in zwei Teilfiguren zwei mögliche Ausführungsformen der Meshmembran des erfindungsgemäßen Aerosolerzeugers in Draufsicht.

In Teilfigur A ist ein Version mit acht runden Verklebeöffnungen 31 gezeigt, welche umfänglich gleichmäßig über den zur flächigen Auflage auf der Trägerstruktur ebenen Außenbereich der einen kreisrunden Grundriss aufweisenden Mesh-Membran 3 verteilt sind, also einen Winkelabstand von 45 Grad zwischen benachbarten Öffnungen 31 aufweisen. Die Verklebeöffnungen in dieser möglichen Ausführungsform liegen also auf einem konzentrischen Kreis um den Mittelpunkt der Mesh-Membran 3 und bilden zusammengenommen ein regelmäßiges Achteck. Die konkrete Zahl der Verklebeöffnungen hat keine Bedeutung und dient nur der Illustration. Eine höhere oder niedrigere Zahl wäre gleichermaßen denkbar, solange mindestens zwei oder mehr, bevorzugt drei oder mehr Verklebeöffnungen vorhanden sind. Auch der hier gezeigte kreisrunde Querschnitt der Verklebeöffnungen hat nur insoweit einen technischen Vorteil, als sich solche Öffnungen leicht herstellen lassen, etwa durch Bohren und somit auch nachträglich in eine anderweitig hergestellte Mesh-Membran eingefügt werden können. Werden jedoch mittels Guss- oder elektro-galvanischer Abscheideverfahren hergestellte Mesh-Membranen eingesetzt, so könnten gleichermaßen Verklebeöffnungen mit anderen Querschnitten, etwa quadratisch, polygonal oder kreisringsegmentförmig verwendet werden.

Teilfigur B zeigt eine Ausführungsform mit auf zwei konzentrischen Ringen um den Mittelpunkt der Mesh-Membran 3 angeordneten Verklebeöffnungen 31, wobei auf jedem der beiden Ringe jeweils vier Verklebeöffnungen 31 liegen. Die Öffnungen innerhalb eines Ringes haben einen Winkelabstand von 90 Grad zwischen Nachbarn und die Öffnungen der beiden Ringe sind um 45 Grad zueinander versetzt.

Durch verschieden Kombinationen von Ringradien und Anzahlen von Verklebeöffnungen pro Ring lassen sich die mechanischen Kopplungseigenschaften der Mesh-Membran an die Trägerstruktur und damit die Schwingungseigenschaften und die Form der sich bei Schwingung ausbildenden stehenden Welle beeinflussen. Hierdurch kann die Tröpfchengrößenverteilung in gewissem Rahmen wie gewünscht eingestellt werden und etwa hinsichtlich einem möglichst hohen Anteil lungenbläschengängiger Tröpfchen (d.h. kleiner 5 Mikrometer) optimiert werden.

**Figur 3** illustriert die Herstellung eines erfindungsgemäßen Aerosolerzeugers in drei Teilansichten.

Teilfigur A illustriert die Schritte a) bis c) des erfindungsgemäßen Herstellungsverfahrens, also das Bereitstellen der Komponenten die die Piezokeramik umfassende Trägerstruktur 2, die Mesh-Membran 3 und den Abschlussring 4, das Einbringen der umfänglichen Verklebeöffnungen 31 (durch einen Bohrer angedeutet) sowie das Aufbringen einer ringförmigen Formation flüssigen Klebstoffs 5' auf der Oberseite der Trägerstruktur 2, wobei das Volumen des Klebstoffs so bemessen ist, dass es unter Berücksichtigung einer etwaigen Volumenänderung während des Aushärtens zumindest dem Volumen aller Verklebeöffnungen plus dem Volumen zweier Klebstoffschichten plus etwaig zu berücksichtigender Verluste entspricht.

Teilfigur B zeigt Herstellungsschritt d) des erfindungsgemäßen Verfahrens, bei dem die Mesh-Membran 3 mit ihrem ebenen Außenbereich auf die Trägerstruktur 2 und dem darauf befindlichen Klebstoff 5' aufgelegt und derart festgedrückt wird, dass der viskose Klebstoff eine möglichst gleichmäßig starke Klebstoffschicht 5a zwischen Trägerstruktur 2 und Mesh-Membran 3 bildet und in die Verklebeöffnungen 31 hinein und durch sie hindurch gedrückt wird und schließlich aus den Öffnungen 31 hervorquellend über die Oberseite der Meshmembran 3 hervorstehende Klebstoffpunkte 5" bildet.

In Teilfigur C sind die letzten beiden Herstellungsschritte, e) und f) dargestellt. In Schritt e) wird zunächst der Abschlussring 4 auf die Oberseite der Mesh-Membran 3 und die darüber hervorragenden Klebstoffpunkte 5' aufgesetzt, wie gewünscht positioniert und dann derart festgedrückt, dass sich eine möglichst gleichmäßige Klebstoffschicht 5b gewünschter Stärke zwischen der Mesh-Membran 3 und dem Abschlussring 4 bildet. Im Schritt f) lässt man nun den 13
Klebstoff aushärten, was die Herstellung des erfindungsgemäßen Aerosolerzeugers abschließt.

### Bezugszeichenliste

- 1: Aerosolerzeuger
- 2: Trägerstruktur/-scheibe
- 20: Öffnung
- 3: Mesh-Membran
- 31: Verklebeöffnung
- 4: Abschlussring
- 5a: untere Klebstoffschicht
- 5b: obere Klebstoffschicht
- 5', 5": flüssiger Klebstoff

## Patentansprüche

1. Aerosolerzeuger zur Verwendung in einem Vernebler zur Aerosolisierung eines Fluides, insbesondere einer wässrigen Wirkstofflösung, umfassend
- eine, insbesondere kreisringförmige, Trägerstruktur (2) mit einer konzentrischen, durchgängigen Öffnung (20),
- eine auf der Trägerstruktur (2) befestigte Mesh-Membran (3), welche die Öffnung (20) abdeckt,
- wobei der Aerosolerzeuger (1) weiterhin einen auf der zur Trägerstruktur (2) abgewandten Oberseite der Mesh-Membran (3) angeordneten Abschlussring (4) aufweist,
- die Mesh-Membran (3) umfänglich über mindestens zwei von ihrer Oberseite zu einer der Trägerstruktur (2) zugewandten Unterseite durchgängige Verklebeöffnungen (31) verfügt, welche gleichmäßig über den Umfang der Mesh-Membran (3) verteilt und durch ausgehärtete Klebstoffsäulen (51) durchgriffen sind, und
- die Klebstoffsäulen (51) eine unterhalb der Mesh-Membran (3) gelegene, mit der der Mesh-Membran (3) zugewandten Oberseite der Trägerstruktur (2) verklebte Klebstoffschicht (5b) mit einer oberhalb der Mesh-Membran (3) gelegene, mit einer der Mesh-Membran (3) zugewandten Unterseite des Abschlussrings (4) verklebte Klebstoffschicht (5a) mechanisch verbinden,
**dadurch gekennzeichnet, dass**
die Mesh-Membran (3) selbst nicht verklebt ist, sondern eine Krafteinleitung von einer Piezo-Keramik der Trägerstruktur (2) in die Mesh-Membran (3) ausschließlich über die die Verklebeöffnungen (31) durchgreifenden Klebstoffsäulen (51) erfolgt.

2. Aerosolerzeuger nach Anspruch 1, **dadurch gekennzeichnet, dass** die Mesh-Membran (3) aus einem nicht mittels eines für den Kontakt mit Wirkstofflösungen zugelassenen Klebstoffs verklebbaren Material, insbesondere Nickel oder einem Fluor-enthaltenden Kunststoff besteht.

3. Aerosolerzeuger nach Anspruch 1 oder 2, **gekennzeichnet durch** drei oder mehr Verklebeöffnungen (31) in der Mesh-Membran (3).

4. Aerosolerzeuger nach einem der Ansprüche 1-3, **dadurch gekennzeichnet, dass** die Mesh-Membran (3) einen kreisrunden Grundriss hat.

5. Aerosolerzeuger nach einem der Ansprüche 1 - 4, wobei die Verklebeöffnungen (31) auf genau einem oder auf genau zwei Kreisen um den Mittelpunkt der Öffnung (20) der Trägerstruktur (2) angeordnet sind.

6. Aerosolerzeuger nach einem der Ansprüche 1 - 5, **dadurch gekennzeichnet, dass** die Trägerstruktur (2) eine Stahlscheibe mit einer auf der der Mesh-Membran (3) abgewandten Unterseite angebrachten kreisringförmigen Piezokeramik ist.

7. Aerosolerzeuger nach einem der Ansprüche 1 - 5, **dadurch gekennzeichnet, dass** die Trägerstruktur eine kreisringförmige Piezokeramik ist.

8. Aerosolerzeuger nach einem der Ansprüche 1 - 7, **dadurch gekennzeichnet, dass** die Verklebeöffnungen (31) rund sind und der Abstand ihres Mittelpunktes vom Rand der Mesh-Membran (3) nicht weniger als das Anderthalbfache ihres Radius beträgt.

9. Aerosolerzeuger nach einem der Ansprüche 1 - 8, **dadurch gekennzeichnet, dass** ein Klebstoff auf Epoxidharzbasis verwendet ist.

10. Verfahren zur Herstellung eines Aerosolerzeugers nach einem der Ansprüche 1 -- 9,
**gekennzeichnet durch** die Schritte
(a) Bereitstellen einer Mesh-Membran (3), eines kreisringförmigen Abschlussrings (4) und einer kreisringförmigen Trägerstruktur (2) mit einer konzentrischen durchgängigen Öffnung (20),
(b) Einbringen von mindestens zwei umfänglich gleichmäßig entlang des Außenrandes der Mesh-Membran (3) angeordneten Verklebeöffnungen (31) in einem gewünschten Muster,
(c) Aufbringen eines Klebstoffringes (5'), welche die Öffnung (20) umschließt auf einer Oberseite der Trägerstruktur (2), wobei das gesamte Klebstoffvolumen des Klebstoffringes (5') etwas größer bemessen ist, als das Gesamtvolumen aller Verklebeöffnungen (31) der Mesh-Membran (3) zuzüglich dem Volumen zweier Klebstoffschichten gewünschter Stärke und Ausdehnung oberhalb bzw. unterhalb der Mesh-Membran (3)
(d) Auflegen und Festdrücken der Mesh-Membran(31), so dass der noch viskose Klebstoff eine möglichst gleichmäßig dicke, zwischen Trägerstruktur (2) und Mesh-Membran (3) gelegene untere Klebstoffschicht (5a) bildet und in die Verklebeöffnungen (31) hinein und durch sie hindurch gedrückt wird, und über die der Trägerstruktur (2) abgewandten Oberseite der Mesh-Membran (3) hinausragende Klebstoffpunkte (5") bildet,
(e) Auflegen und Festdrücken des Abschlussrings (4), so dass eine möglichst gleichmäßig dicke, zwischen Mesh-Membran (3) und Abschlussring (4) gelegene obere Klebstoffschicht (5b) gebildet wird, und
(f) Aushärtenlassen des Klebstoffs,
wobei die Mesh-Membran (3) selbst nicht verklebt wird, so dass eine Krafteinleitung von einer Piezo-Keramik der Trägerstruktur (2) in die Mesh-Membran (3) ausschließlich über die die Verklebeöffnungen (31) durchgreifenden Klebstoffsäulen (51) erfolgen kann.

11. Verfahren nach dem vorhergehenden Anspruch, **dadurch gekennzeichnet, dass** nach dem letzten Schritt eine Nachbehandlung stattfindet, bei der überstehende Klebstoffreste entfernt werden.

## Claims

1. An aerosol generator for use in a nebulizer for aerosolizing a fluid, in particular an aqueous drug solution, comprising:
- a support structure (2), in particular annular, with a concentric, continuous opening (20);
- a mesh membrane (3) attached to the support structure (2), which covers the opening (20);
- the aerosol generator (1) further comprising a termination ring (4) arranged on the upper surface of the mesh membrane (3) facing away from the support structure (2);
- the mesh membrane (3) having at least two continuous adhesive openings (31) extending from its upper surface to a lower surface facing the support structure (2), which are evenly distributed around the circumference of the mesh membrane (3) and penetrated by cured adhesive columns (51); and
- the adhesive columns (51) mechanically connect an adhesive layer (5b) located below the mesh membrane (3) and bonded with the upper side of the support structure (2) facing the mesh membrane (3) to an adhesive layer (5a) located above the mesh membrane (3) and bonded to the underside of the end ring (4) facing the mesh membrane (3),
**characterized in that**
the mesh membrane (3) itself is not bonded, but rather a force is introduced from a piezo-ceramic of the support structure (2) into the mesh membrane (3) exclusively via the adhesive columns (51) extending through the bonding openings (31).

2. The aerosol generator according to claim 1, **characterized in that** the mesh membrane (3) consists of a material that cannot be bonded by means of an adhesive approved for contact with drug solutions, in particular nickel or a fluorine-containing plastic.

3. The aerosol generator according to claim 1 or 2, **characterized by** three or more bonding openings (31) in the mesh membrane (3).

4. The aerosol generator according to one of claims 1-3, **characterized in that** the mesh membrane (3) has a circular plan.

5. The aerosol generator according to one of claims 1 to 4, wherein the bonding openings (31) are arranged on exactly one or on exactly two circles around the center of the opening (20) of the support structure (2).

6. The aerosol generator according to one of claims 1 - 5, **characterized in that** the support structure (2) is a steel disc with an annular piezoelectric ceramic element mounted on the underside facing away from the mesh membrane (3).

7. The aerosol generator according to one of claims 1 - 5, **characterized in that** the support structure is an annular piezoceramic.

8. The aerosol generator according to one of claims 1 - 7, **characterized in that** the bonding openings (31) are round and the distance of their center point from the edge of the mesh membrane (3) is not less than one and a half times their radius.

9. The aerosol generator according to one of claims 1 - 8, **characterized in that** an epoxy resin-based adhesive is used.

10. A Method for manufacturing an aerosol generator according to one of claims 1-9, **characterized by** the steps:
(a) providing a mesh membrane (3), an annular end ring (4), and an annular support structure (2) with a concentric continuous opening (20);
(b) introducing at least two bonding openings (31) arranged uniformly around the outer edge of the mesh membrane (3) in a desired pattern;
(c) applying an adhesive ring (5') surrounding the opening (20) to a top surface of the support structure (2), wherein the total adhesive volume of the adhesive ring (5') is slightly larger than the total volume of all bonding openings (31) of the mesh membrane (3) plus the volume of two adhesive layers of desired thickness and extent above and below the Mesh membrane (3)
(d) Applying and pressing down the mesh membrane (31) so that the still viscous adhesive forms a lower adhesive layer (5a) of as uniform thickness as possible between the support structure (2) and the mesh membrane (3), and is pressed into and through the bonding openings (31), forming adhesive dots (5") projecting beyond the upper surface of the mesh membrane (3) facing away from the support structure (2),
(e) Applying and pressing down the end ring (4) so that an upper adhesive layer (5b) of as uniform thickness as possible is formed between the mesh membrane (3) and the end ring (4), and
(f) Allowing the adhesive to cure,
wherein the mesh membrane (3) itself is not bonded, so that force transmission from a piezoelectric ceramic of the support structure (2) into the mesh membrane (3) occurs exclusively via the adhesive columns (51) passing through the bonding openings (31).

11. Method according to the preceding claim, **characterized in that** after the last step a post-treatment takes place in which excess adhesive residues are removed.

## Revendications

1. Générateur d'aérosol pour nébuliseur destiné à l'aérosolisation d'un fluide, notamment d'une solution médicamenteuse aqueuse, comprenant :
- une structure de support (2), notamment annulaire, présentant une ouverture continue et concentrique (20);
- une membrane en maille (3) fixée à la structure de support (2) et recouvrant l'ouverture (20);
- le générateur d'aérosol (1) comprenant en outre un anneau de terminaison (4) disposé sur la face supérieure de la membrane en maille (3), opposée à la structure de support (2);
- la membrane en maille (3) présentant au moins deux ouvertures de collage continues (31) s'étendant de sa face supérieure à sa face inférieure, face à la structure de support (2), réparties uniformément sur la circonférence de la membrane en maille (3) et traversées par des colonnes adhésives polymérisées (51), et
- les colonnes adhésives (51) relient mécaniquement une couche adhésive (5b) située sous la membrane (3) et collée à la face supérieure de la structure de support (2) faisant face à la membrane en maille (3) à une couche adhésive (5a) située au-dessus de la membrane en maille (3) et collée à la face inférieure de l'anneau d'extrémité (4) faisant face à la membrane en maille (3),
**caractérisé en ce que**
la membrane en maille (3) elle-même n'est pas collée, mais qu'une force est introduite dans la membrane en maille (3) par un élément piézo-céramique de la structure de support (2) exclusivement via les colonnes adhésives (51) traversant les ouvertures de collage (31).

2. Le générateur d'aérosol selon la revendication 1, **caractérisé en ce que** la membrane en maille (3) est constituée d'un matériau qui ne peut être collé au moyen d'un adhésif autorisé pour le contact avec des solutions médicamenteuses, notamment le nickel ou un plastique fluoré.

3. Le générateur d'aérosol selon la revendication 1 ou 2, **caractérisé par** trois ouvertures de collage (31) ou plus dans la membrane en maille (3).

4. Le générateur d'aérosol selon l'une des revendications 1 à 3, **caractérisé en ce que** la membrane en maille (3) présente une forme circulaire.

5. Le générateur d'aérosol selon l'une des revendications 1 à 4, dans lequel les ouvertures de collage (31) sont disposées sur exactement un ou exactement deux cercles autour du centre de l'ouverture (20) de la structure de support (2).

6. Le générateur d'aérosol selon l'une des revendications 1 à 5, **caractérisé en ce que** la structure de support (2) est un disque en acier avec un élément céramique piézoélectrique annulaire monté sur la face inférieure opposée à la membrane en maille (3).

7. Le générateur d'aérosol selon l'une des revendications 1 à 5, **caractérisé en ce que** la structure de support est une structure annulaire piézocéramique.

8. Le générateur d'aérosol selon l'une des revendications 1 à 7, **caractérisé en ce que** les ouvertures de collage (31) sont rondes et que la distance de leur centre au bord de la membrane en maille (3) est au moins égale à une fois et demie leur rayon.

9. Le générateur d'aérosol selon l'une des revendications 1 à 8, **caractérisé en ce qu'**un adhésif à base de résine époxy est utilisé.

10. Procédé de fabrication d'un générateur d'aérosol selon l'une des revendications 1 à 9, **caractérisé par** les étapes suivantes :
(a) fourniture d'une membrane à mailles (3), d'un anneau d'extrémité annulaire (4) et d'une structure de support annulaire (2) comportant une ouverture continue concentrique (20);
(b) réalisation d'au moins deux ouvertures de collage (31) disposées uniformément sur le pourtour extérieur de la membrane à mailles (3) selon un motif souhaité;
(c) Application d'un anneau adhésif (5') entourant l'ouverture (20) sur la surface supérieure de la structure de support (2), le volume total d'adhésif de l'anneau (5') étant légèrement supérieur au volume total de toutes les ouvertures de collage (31) de la membrane (3), augmenté du volume de deux couches adhésives d'épaisseur et d'étendue souhaitées, placées au-dessus et au-dessous de la membrane (3).
(d) Appliquer et presser la membrane (31) de manière à ce que l'adhésif encore visqueux forme une couche inférieure (5a) d'épaisseur aussi uniforme que possible entre la structure de support (2) et la membrane (3), et pénètre dans les ouvertures de collage (31), formant ainsi des points d'adhésif (5") dépassant de la surface supérieure de la membrane (3) et opposés à la structure de support (2).
(e) Appliquer et presser l'anneau d'extrémité (4) de manière à former une couche supérieure (5b) d'épaisseur aussi uniforme que possible entre la membrane (3) et l'anneau d'extrémité (4).
(f) Laisser l'adhésif sécher,
dans laquelle la membrane en maille (3) n'est pas collée, de sorte que la transmission de la force de la céramique
piézoélectrique de la structure de support (2) à la membrane en maille (3) s'effectue exclusivement par l'intermédiaire des colonnes adhésives (51) traversant les ouvertures de collage (31).

11. Procédé selon la revendication précédente, **caractérisé en ce qu'**après la dernière étape, un post-traitement est effectué pour éliminer les résidus d'adhésif excédentaires.
